# EUROPÄISCHE PATENTSCHRIFT

(11) **EP 4 120 980 B1**
(45) Veröffentlichungstag und Bekanntmachung des Hinweises auf die Patenterteilung: **22.10.2025**
(21) Anmeldenummer: 21716602.4
(22) Anmeldetag: 16.03.2021
(51) Int. Cl.: A61F 13/15, A61F 13/551, B65D 85/00

(54) **VERFAHREN ZUM UMHÜLLEN EINES HYGIENEARTIKELS ODER EINES STAPELS AUS EINER ANZAHL VON HYGIENEARTIKELN**
METHOD FOR WRAPPING A SANITARY ITEM OR A STACK COMPRISING A NUMBER OF SANITARY ITEMS
PROCÉDÉ D'EMBALLAGE D'UN ARTICLE SANITAIRE OU D'UN EMPILEMENT COMPRENANT UN CERTAIN NOMBRE D'ARTICLES D'HYGIÈNE

(30) Priorität: 16.03.2020 DE 102020203360
(43) Veröffentlichungstag der Anmeldung: 25.01.2023
(73) Patentinhaber: BW Converting GmbH, 56564 Neuwied (DE)
(72) Erfinder: ALLAR, Andrea, 56170 Bendorf (DE); BARZ, Heinz, 56751 Einig (DE)
(74) Vertreter: Tergau & Walkenhorst Intellectual Property GmbH
(86) Internationale Anmeldenummer: PCT/EP2021/056729
(87) Internationale Veröffentlichungsnummer: WO 2021/185866

(56) Entgegenhaltungen:
- EP-A1- 1 905 693
- DE-A1- 102011 114 152
- DE-U1- 202016 001 372
- US-A1- 2009 324 917

## Beschreibung

Die Erfindung bezieht sich auf ein Verfahren zum Umhüllen eines Hygieneartikels oder eines Stapels aus einer Anzahl von Hygieneartikeln.

Die DE 10 2011 114152 A1 bezieht sich auf ein Verfahren zum Verpacken von Zeitschriften, Werbeprospekten oder Briefen (Kuvertierung). Die DE 20 2016 001372 U1 offenbart eine Verpackung mit Hygienetüten aus recyclebarem Material für benutzte Papiertaschentücher. Die US 2009/0324917 A1 bezieht sich auf die Verpackung von Hygieneartikeln unter Verwendung einer biologisch abbaubaren Folie aus thermoplastischer Stärke und Polymilchsäure. Das Material und dessen Herstellung sind detailliert beschrieben, nicht jedoch das Verpackungsverfahren. WO 2004/113061 A1 beschreibt die Verpackung fester Produkte, insbesondere von Seifenstücken. US 2006/207224 A1 beschreibt die Herstellung von Zigaretten- bzw. Zigarrenschachteln. US 2015/114866 A1 beschreibt einen Umhüllungsrohling, der mit einer Prägung oder einem Sichtfenster versehen ist.

Unter "Hygieneprodukt" oder "Hygieneartikel" sind im Folgenden und im Sinne der vorliegenden Anmeldung insbesondere absorbierende Einwegartikel zur Aufnahme von Körperausscheidungen zu verstehen. Solche Hygiene-Einmal- oder Wegwerfprodukte ("Disposable Hygiene Products") werden üblicherweise einzeln oder in Produktstapeln verpackt angeboten und verkauft. Die vorliegend insbesondere in Betracht gezogenen Hygieneprodukte umfassen insbesondere Damenbinden, Windeln, Inkontinenzprodukte, Slipeinlagen, Meatpads, Petpads (für Haustiere), Nursing Pads oder dergleichen. Derartige Produkte, insbesondere in ihrer Ausgestaltung als Einmal- oder Wegwerfprodukte, sind üblicherweise aus einem saugfähigen Material ausgeführt und darüber hinaus mit einem Saugkern versehen, der aufgenommene Flüssigkeit beispielsweise in ein Gel oder dergleichen umwandelt. Diese Produkte weisen insbesondere unter anderem einen vergleichsweise weichen, elastischen und verformbaren Grundkörper auf. Die Verpackung oder Umhüllung derartiger Produkte ist daher einerseits aus hygienischen Gründen grundsätzlich wichtig, andererseits aber auch funktionsbedingt wünschenswert, um vor dem eigentlichen Gebrauch des Hygieneartikels das Eindringen von Feuchtigkeit weitgehend zu reduzieren.

Die Verpackung oder Umhüllung derartiger Produkte, einzeln oder als Produktstapel mit einer geringen Anzahl von Einzelprodukten, erfolgt üblicherweise im Hinblick auf die Erfordernisse des automatisierten Verpackungsprozesses mit einer strapazierfähigen, elastisch dehnbaren Folie auf Kunststoff- oder Plastikbasis. Diese wird üblicherweise als Endlosmaterial auf einer Rolle oder dergleichen bereitgestellt und gemeinsam mit den zu verpackenden Produkten oder Produktstapeln einer Umhüllungsstation zugeführt. Dort wird aus der zugeführten Folie ein den Produktstrom umgebender Folienschlauch gebildet, und anschließend werden die Umhüllungen vereinzelt und die Stirnseiten verschweißt, so dass die jeweiligen Produkte vollständig umschließende Umhüllungen entstehen.

Es besteht nunmehr der Wunsch, unter anderem aus Gründen der Nachhaltigkeit und des Umweltschutzes für derartige umhüllte Produkte einen Materialwechsel des Hüllmaterials hin zu einem nachhaltigen, vorzugsweise biologisch abbaubaren oder recycelbaren Rohstoff vorzunehmen. Die Hygieneprodukte selbst können nämlich, gerade auch im Hinblick auf die vorgesehene Ausgestaltung als Einweg- oder Wegwerf-Produkt, aus einem geeignet gewählten, nachhaltigen, biologisch abbaubaren Material beispielsweise auf Faserbasis hergestellt werden, für ein hochwertiges, nachhaltiges und biologisch abbaubares Gesamtensemble wäre es somit wünschenswert, auch das Hüllmaterial hieran geeignet anzupassen. Prozessbedingt und im Hinblick auf die gängigen, in der Regel automatisierten Abläufe beim Verpacken von Hygieneprodukten sind die gängigen Umhüllungsverfahren aber nicht für die Verwendung derartiger, in der Regel nicht dehnbarer Materialien wie beispielsweise Papier geeignet.

Der Erfindung liegt daher die Aufgabe zugrunde, ein Verfahren zum Umhüllen eines Hygieneartikels oder eines Stapels aus einer Anzahl von Hygieneartikeln anzugeben, mit dem die Verpackung des Hygieneartikels bzw. des Stapels aus Hygieneartikeln in einer aus einem nachhaltigen, insbesondere biologisch abbaubaren und/oder recycelbaren, Rohstoff gebildeten Umhüllung ermöglicht wird, wobei die Aufbewahrung feucht gehaltener Produkte oder auch die trockene Aufbewahrung von Hygieneprodukten in feuchter Außenumgebung unterstützt werden soll.

Diese Aufgabe wird erfindungsgemäß gelöst, durch ein Verfahren mit den Merkmalen des Anspruchs 1.

Vorteilhafte Ausgestaltungen der Erfindung sind Gegenstand der Unteransprüche.

Die Erfindung geht von der Überlegung aus, dass ein wesentlicher Hinderungsgrund bei der Umstellung des Umhüllungsmaterials auf die erwünschten nachhaltigen Rohstoffe in den Prozessabläufen der gängigen Verpackungsverfahren für Hygieneprodukte zu sehen sein dürfte. Diese gängigen Prozessabläufe bringen nämlich eine Reihe von Erfordernissen an die Materialeigenschaften der bearbeiteten Verpackungsmaterialien mit sich. Insbesondere setzen die gängigen Verfahren eine ausreichende Rissfestigkeit und ggf. eine gewisse Elastizität und Dehnbarkeit des Hüllmaterials voraus, die für die als Rohstoff nunmehr vorgesehenen nachhaltigen, biologisch abbaubaren Materialien unter Umständen nicht gegeben sind. Um dem entgegenzuwirken, ist daher vorliegend eine grundsätzliche Prozessumstellung beim Verpacken oder Umhüllen der Hygieneprodukte vorgesehen. Hierzu sollte auf Elemente beispielsweise aus dem Bereich der Briefumschlagtechnologie zurückgegriffen werden, die beispielsweise mit vergleichsweise hohen Takt- und Durchsatzraten die Verpackung von individualisierten Sendungen in Briefumschlägen aus Papier ermöglicht.

Eine Berücksichtigung der Materialeigenschaften der erwünschten Hüllmaterialien auch bei weitgehend gering gehaltenen Kosten ist dabei insbesondere erreichbar, indem - analog zu entsprechenden Verfahren bei der Kuvertierung von zu versendenden Güter - sowohl die zu verpackenden Güter als auch die Verpackungen vor Ort hergestellt werden können. Für eine möglichst flexible und individuell anpassbare Umhüllung können die Verpackungen insbesondere nicht bereits vor der Zusammenführung mit dem zu umhüllenden Produkt hergestellt, sondern zunächst nur Umhüllungsrohlinge bereitgestellt werden. Diese Umhüllungsrohlinge werden mit dem Produkt oder Produktstapel vereint, wobei gerade im Hinblick auf die angestrebte Materialkombination eines vergleichsweise weichen Produkts (typisch für Hygieneprodukte) und eines vergleichsweise harten Hüllmaterials (typisch für Papier als angestrebtes Hüllmaterial) zunächst eine positionsgenaue Ablage des Produkts oder Produktstapels auf dem Umhüllungsrohling erfolgen sollte. Erst anschließend wird das Produkt oder der Produktstapel durch Faltung des Umhüllungsrohlings umhüllt.

Ebenfalls im Hinblick auf die angestrebte Materialkombination sollte der Umhüllungsrohling geeignet für die nachfolgende Faltung vorbereitet werden. Dabei sollte berücksichtigt werden, dass im Hinblick auf die erwartete Dicke der Produkte oder Produktstapel bei der Umhüllung eine umlaufende Seitenkante notwendig ist. Die Produkte können dabei, abhängig von der jeweils vorgegebenen Spezifikation für das Endprodukt, ungefaltet oder auch gefaltet vorliegen und verpackt werden, so dass bereits aus diesem Grunde unterschiedliche Dicken im Produktspektrum vorliegen können. Um diese zuverlässig und reproduzierbar bereitzustellen, sollten vor der Vereinigung von Umhüllungsrohling und Produkt geeignete Falzkanten in den Rohling eingebracht werden, die in der Art einer vorgebbaren Solllinie bei der anschließenden Faltung Lage und Ausrichtung der Knickkante definieren.

Das für die Bildung der Umhüllung vorgesehene Material ist ganz aus nachhaltigem, insbesondere biologisch abbaubarem und/oder recycelbarem Rohstoff gebildet, der vorzugsweise kein Mikroplastik erzeugt. Als besonders geeignet und somit bevorzugt werden hierfür angesehen Papier (gestrichen oder ungestrichen), Kraftpapier, fettdichtes und/oder nassfestes Papier, Filterpapier, Krepp-Papier, Pergamid, Eukalyptus, Tissue, Bambus, Papyrus, Hanf, Filz, Wolle, Baumwolle, Leinen oder andere derartige Stoffe.

Das für die Bildung der Umhüllung vorgesehene Material kann einlagig oder mehrlagig sein. Mehrlagiges Material ist vorzugsweise gebildet von einer Basisschicht, die mit einer Schicht anderen, biologisch abbaubaren Materials kaschiert ist. Das Material kann je nach Produktvorgabe glatt oder vorgeprägt sein oder inline geprägt werden.

Das für die Umhüllung vorgesehene Material kann geschlossene oder teilweise offene Oberfläche haben, z. B. aufgrund faseriger Eigenschaften oder aufgrund von einer gezielt eingebrachten Perforation. Eine derartige, teilweise offene Oberfläche kann besonders vorteilhaft sein um sicherzustellen, dass sich die Umhüllung nicht unter Druck im weiteren Prozessverlauf aufgrund von im Päckcheninneren eingeschlossener Luft wieder öffnet.

Bevorzugt weist das für die Umhüllung vorgesehene Material eine Grammage oder ein Papiergewicht von 25 - 80 gsm auf.

Der Umhüllungsrohstoff kann dem Prozess als Endlosbahn von einer Rolle oder auch einzeln als Blatt oder Bogen zugeführt werden. Im Falle der Zuführung aus einer Endlosbahn wird der Umhüllungsrohling vorteilhafterweise vor der Zuführung zur Umhüllungsstation oder auch nach der Vereinigung mit dem Produkt oder Produktstapel und einer ggf. vorgesehenen Faltung der Seitenklappen aus der Endlosbahn vereinzelt, wobei an geeigneter Stelle im Prozess in weiterer vorteilhafter Ausgestaltung ein Querschneiden des Umhüllmaterials zwischen zwei Produkten zur Trennung und zur Erzeugung der vorderen und rückseitigen Laschen vorgesehen ist.

Vorteilhafterweise werden die Seitenkanten des Umhüllungsrohlings zur Erzeugung der seitlichen Umhüll-Laschen vor oder in der Umhüllungsstation zugeschnitten.

Prozessgemäß ist vorgesehen, dass nach vollständiger Umhüllung des Produkts oder Produktstapels, also nach dem Einfalten sämtlicher Umhüll-Laschen, die vorder- und rückseitigen Laschen an den Seitenlaschen und/oder aneinander fixiert werden. Vorteilhafterweise und für einen besonders günstigen und zuverlässigen Prozessablauf werden die Laschen der Umhüllung dabei in oder nach der Umhüllungsstation thermisch und/oder mechanisch und/oder durch Kleben, vorzugsweise wiederverschließbar, aneinander fixiert. Das Verschließen der Umhüllung oder das Fixieren erfolgt dabei besonders bevorzugt mittels adhäsivem Fixiermaterial, z. B. Heißleim, Kaltleim, selbstklebendem Tape oder dergleichen, mittels mechanischem Fixiermaterial, wie beispielsweise Klettverschluss, Druckknopf oder dergleichen, mittels thermoplastischem Fixiermaterial, beispielsweise heiß aktivierbarem Kaltleim, Wachs, Lack oder dergleichen, und/oder ohne separates Fixiermaterial, beispielsweise durch rein mechanisches oder thermomechanisches Verprägen, Ultraschall, Einschieben der Laschen in dafür vorgesehene Schlitze oder dergleichen.

Die verwendeten Fixiermaterialien oder -methoden können vollflächig oder in Teilbereichen der Umhüllung aufgebracht werden, beispielsweise bereits durch den Materialhersteller oder inline in der Maschine. Dazu ist in der Verpackungsmaschine vorzugsweise ein geeignetes Modul vorgesehen, mit dem das Fixiermaterial geeignet auf die ausgewählten Zonen oder Teilbereiche aufgebracht werden kann, vorzugsweise durch Drucken, Sprühen oder andere für das betreffende Material geeignete Verfahren.

In ganz besonders bevorzugter Ausgestaltung ist eine Personalisierung oder Individualisierung des umhüllten Hygieneprodukts oder -produktstapels vorgesehen oder ermöglicht, indem der Hüllenrohling mit einer Bedruckung versehen wird. Die Bedruckung kann dabei in Form einzelner Elemente oder Ornamente oder alternativ auch vollflächig, innen- und/oder außenseitig, aufgebracht werden. Eine Bedruckung mit einzelnen Elementen ist dabei insbesondere für Verwender- oder Adressaten-spezifische Informationen vorteilhaft, wohingegen eine vollflächige Bedruckung vorteilhafterweise beispielsweise vorgesehen sein kann, um die Umhüllung undurchsichtig oder intransparent zu machen. Dieser Aspekt könnte beispielsweise bei an sich durchscheinenden oder transparenten Hüllmaterialien wie Pergament oder dergleichen besonders vorteilhaft sein. Mehrere solcher Bedruckungen können auch in Kombination miteinander vorgesehen sein.

Dabei kann insbesondere ein geeignet ausgestaltetes Bedruckungsmodul in die Verpackungsmaschine integriert sein, mit dem beispielsweise hersteller- oder auch konsumentenspezifische Informationen oder Gestaltungselemente auf die Umhüllung aufgebracht werden können.

In alternativer oder zusätzlicher Ausgestaltung wird der Hüllenrohling mit einer Prägung versehen. Die Prägung kann dabei in der Art eines Motivs oder alternativ auch als vollflächige Prägung ausgestaltet sein, beispielsweise um der Umhüllung eine erwünschte Textur und/oder eine besondere Haptik zu verleihen. Dies kann analog zur Bedruckung vornehmlich gestalterischen Zwecken dienen, beispielsweise in Form der Aufbringung eines Herkunftshinweises in der Art eines Siegels oder Wasserzeichens, oder auch technischen Zwecken, beispielsweise um mittels einer solchen Prägung eine mechanische Verzahnung oder Verbindung benachbarter oder aufeinanderliegender Teile der Umhüllung zu bewirken.

In alternativer oder zusätzlicher vorteilhafter Ausgestaltung wird der Hüllenrohling mit einer Perforierung oder Perforation versehen. Diese kann, zusätzlich zu oder anstelle von dekorativen Zwecken, beispielsweise zur Vorgabe einer Ab- oder Aufrisskante zum späteren Öffnen der Umhüllung mit definierter Entnahmeöffnung und/oder auch als Entlastungsperforation zur Vermeidung eines unerwünschten Druckaufbaus oder der unerwünschten Ausbildung eines Luftpolsters innerhalb der Umhüllung dienen.

In ganz besonders vorteilhafter Ausgestaltung wird beim Hüllenrohling ein Fenster ausgeschnitten, das anschließend durch Einkleben mit einer Fensterfolie verschlossen wird. Als Material für die Fensterfolie ist vorzugsweise ein geeignet transparentes Material wie beispielsweise Pergament vorgesehen, das im Hinblick auf die angestrebten Nachhaltigkeitsaspekte geeignet mit dem eigentlichen Hüllmaterial harmoniert. Damit ist auch bei der Verwendung an sich undurchsichtiger Hüllmaterialien wie beispielsweise Papier eine Ausgestaltung der Umhüllung ermöglicht, bei der der Benutzer unmittelbar das in der Umhüllung befindliche Produkt und ggf. dessen Zustand in Augenschein nehmen kann.

Vorteilhafterweise wird der Hygieneartikel bzw. der Stapel aus Hygieneartikeln einerseits und ein zugeordneter Umhüllungsrohling andererseits getaktet und zueiander synchronisiert der Umhüllungsstation zugeführt. In besonders vorteilhafter Weiterbildung wird dabei die Taktung oder Synchronisierung von Hygieneartikel bzw. Stapel aus Hygieneartikeln einerseits und zugeordnetem Umhüllungsrohling andererseits durch Abfragen einer Druckmarke überwacht, so dass die korrekte Zuordnung des Produkts zur ihm zugeordneten Umhüllung geeignet überwacht und auch die positionsgerechte Ablage des Produkts auf dem Umhüllungsrohling sichergestellt werden kann. Die Druckmarke kann dabei an einer im fertigen Produkt sichtbaren oder auch nicht sichtbaren Position angebracht sein.

In weiterer vorteilhafter Ausgestaltung wird der oder werden die Hygieneartikel nach dem Einfalten in die Umhüllung an dieser fixiert. Gerade in Kombination mit einem in die Umhüllung eingebrachten Sichtfenster kann dadurch sichergestellt werden, dass ein beispielsweise am Produkt angebrachter oder aufgedruckter Informationsbereich für den Verwender auch tatsächlich einsehbar ist und auch bei Faltung oder sonstiger mechanischer Beanspruchung des umhüllten Produkts erhalten bleibt. Eine solche Fixierung kann insbesondere dann besonders vorteilhaft sein, wenn das Produkt eine Damenbinde ist. Eine solche Damenbinde kann mit einem Silikonstreifen als Abdeckung für einen Klebebereich versehen sein, wobei der Kleber nach Abziehen dieses Streifens dann am Produkt verbleibt. Daher ist bei der Einzelverpackung dieses Produkts in ganz besonders vorteilhafter Ausgestaltung das Verkleben oder Verbinden dieses Silikonstreifens mit dem Umhüllungsmaterial vorgesehen. Damit wird erreicht, dass wird der Silikonstreifen beim Auspacken, also beim Entfernen der Umhüllung, automatisch mit abgezogen wird, so dass die Handhabung für den Verwender entsprechend erleichtert ist. Zudem entsteht bei dieser Ausgestaltung nur ein entsorgungsbedürftiges Abfallteil. Dieses ist im Sinne der Abfall- oder Entsorgungswirtschaft "sortenrein", da es ausschließlich aus Papier gebildet ist.

Ein durch das vorstehend beschrieben Verfahren erhältlicher umhüllter Hygieneartikel, insbesondere ein umhüllter elastischer Ein- oder Mehrwegartikel mit integriertem Absorber zur Aufnahme von Körperflüssigkeiten, versehen mit einer Umhüllung aus einem nachhaltigen, insbesondere biologisch abbaubaren und/oder recycelbaren, Rohstoff, wird ausdrücklich als eigenständig erfinderisch angesehen. Ebenso wird die Verwendung des Umhüllungsverfahrens zum Umhüllen absorbierender Einwegartikel zur Aufnahme von Körperausscheidungen, insbesondere Damenbinden, Slipeinlagen, Inkontinenzvorlagen, Windeln oder Windelhosen für Babies, Erwachsene oder Haustiere, Unterlagen als Betteinlage, Wickelunterlage, Haustierunterlage, Wundauflage, oder Einlage für Nahrungsmittel-Trays (z.B. Fleisch, Fisch), Stilleinlagen, Einwegtaschentücher oder dergleichen als eigenständig erfinderisch angesehen.

Die mit der Erfindung erzielten Vorteile bestehen insbesondere darin, dass durch die konzeptionelle Verwendung von in Kuvertierungs- oder Briefumschlagmaschinen verwendeten Prozesselementen bei der individualisierten Verpackung von Hygieneprodukten, einzeln oder als Stapel, gefaltet oder nicht gefaltet, auch die Verwendung alternativer Umhüllungsmaterialien, insbesondere nachhaltiger, biologisch abbaubarer Rohstoffe, möglich wird, die den eigentlich für Hygieneprodukte gängigen Prozess- und Belastungserfordernissen nicht entsprechen würden. Insbesondere können auch nicht-elastisch verformbare Materialien verarbeitet werden, die für die bisherigen Standardprozesse nicht geeignet sind, weil sie dort Falten schlagen oder einreißen würden. Das damit ermöglichte, besonders angestrebte und als eigenständig erfinderisch angesehene Ersetzen der marktüblichen Plastik-Umhüllungsfolie für Hygieneprodukte durch einen nachhaltigeren Rohstoff bewirkt ein Reduzieren des Plastikeintrags in die Umwelt und ein Reduzieren des insgesamt anfallenden Abfalls durch Einsatz biologisch abbaubaren und/oder recycelbaren Umhüllmaterials.

Falls ein solcher neuartiger Prozess einen bisher marktüblichen "Easypack"-Prozess ersetzt, bei dem die Produkte ohne Silikonpapier direkt auf die Umhüllungsfolie laminiert werden, führt dies zu einer Produktverbesserung durch:
- Optimaler Adhäsivleimtransfer aufs Produkt, ohne Blasenbildung
- Wegfall der kritischen Querverteilung eines breiten Adhäsivleimauftrags
- Wegfall geschwindigkeitslimitierender Schaltzeiten bei mehreren quer applizierten Adhäsivleimstreifen

Ein Ausführungsbeispiel der Erfindung wird anhand einer Zeichnung näher erläutert. Darin zeigen:
- FIG. 1: einen Hygieneartikel unmittelbar vor seiner Verpackung,
- FIG. 2: den Hygieneartikel nach FIG. 1 in teilweise verpacktem Zustand,
- FIG. 3a, 3b: jeweils schematisch den Verfahrensablauf bei der Umhüllung des Hygieneartikels gem. FIG. 1, und
- FIG. 4: einige Varianten von vorgeschnittenen Umhüllungsrohlingen mit eingebrachten Falzkanten.

Gleiche Teile sind in allen Figuren mit denselben Bezugszeichen versehen.

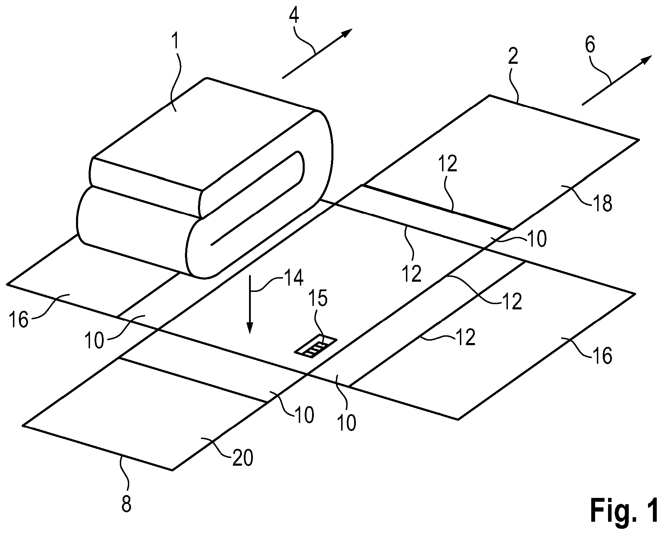

Das Hygieneprodukt 1 oder der Hygieneartikel 1 gemäß FIG. 1, im Ausführungsbeispiel ein absorbierender Einwegartikel zur Aufnahme von Körperausscheidungen, soll in der Art einer Einzelverpackung mit einer Umhüllung 2 versehen werden. Im Ausführungsbeispiel ist das Hygieneprodukt 1 ein gefaltetes Produkt, alternativ kann es aber auch ungefaltet verpackt werden, oder es könnte sich auch um einen Stapel aus einer Anzahl derartiger Hygieneprodukte 1 handeln. Insbesondere kann das Hygieneprodukt 1 eine Damenbinde, eine Windel, ein Inkontinenzprodukt, eine Slipeinlage, ein Meatpad, ein Petpad (für Haustiere), ein Nursing Pad oder dergleichen sein.

Für die Umhüllung 2 ist die Verwendung eines nachhaltigen, biologisch abbaubaren und/oder recycelbaren Rohstoffs als Basismaterial vorgesehen, der vorzugsweise kein Mikroplastik erzeugt. Als besonders geeignet und somit bevorzugt werden hierfür im Ausführungsbeispiel angesehen Papier (gestrichen oder ungestrichen), Kraftpapier, fettdichtes und/oder nassfestes Papier, Filterpapier, Krepp-Papier, Pergamid, Eukalyptus, Tissue, Bambus, Papyrus, Hanf, Filz, Wolle, Baumwolle, Leinen oder andere derartige Stoffe.

Aufgrund der Materialeigenschaften eines solchen Rohstoffs, insbesondere mangelnde Elastizität, höhere Sprödigkeit und vergleichsweise geringere Reißfestigkeit, ist die Verwendung der an sich üblichen Verpackungsverfahren für derartige Hygieneartikel 1 vorliegend nicht geeignet. Es ist daher im Ausführungsbeispiel vorgesehen, den Hygieneartikel 1 unter Verwendung von eigentlich für Kuvertier- oder Briefumschlagmaschinen vorgesehenen Prozesselementen in die Umhüllung 2 einzuschlagen. Dazu wird der Hygieneartikel 1, wie dies in FIG. 1 skizziert ist, zunächst entlang einer durch den Pfeil 4 angedeuteten Transportrichtung einer Umhüllungsstation zugeführt und dort mit einem aus dem nachhaltigen Rohstoff gebildeten, ebenfalls entlang der durch den Pfeil 6 angedeuteten Transportrichtung geförderten Umhüllungsrohling 8 in Überlappung gebracht. Zuvor wurde der Umhüllungsrohling 8 in einer Vorrill-Station mit zur Bildung von Seitenkanten 10 der Umhüllung 2 geeigneten Falzkanten 12 versehen.

**In** der Umhüllungsstation wird der Hygieneartikel 1, wie durch den Pfeil 14 angedeutet, in einer vorgegebenen Sollposition auf dem Umhüllungsrohling 8 positioniert. Die korrekte Zuordnung des Hygieneartikels 1 oder Produkts zum ihm zugeordneten Umhüllungsrohling und auch die positionsgerechte Ablage des Produkts auf dem Umhüllungsrohling 8 werden dabei mittels einer Druckmarke 15 geeignet überwacht. Anschließend wird der Hygieneartikel 1 durch Faltung des Umhüllungsrohlings 8 und anschließendes Verschließen umhüllt, wobei die Seitenlaschen 16 unter Ausbildung der durch die Falzkanten 12 vordefinierten Seitenkanten 10 eingefaltet werden, die vorder- und rückseitigen Laschen 18, 20 unter Ausbildung der durch die für diese vorgesehenen Falzkanten 12 vordefinierten Seitenkanten 10 eingefaltet werden, und schließlich die vorder- und rückseitigen Laschen 18, 20 an den Seitenlaschen 16 und aneinander fixiert werden.

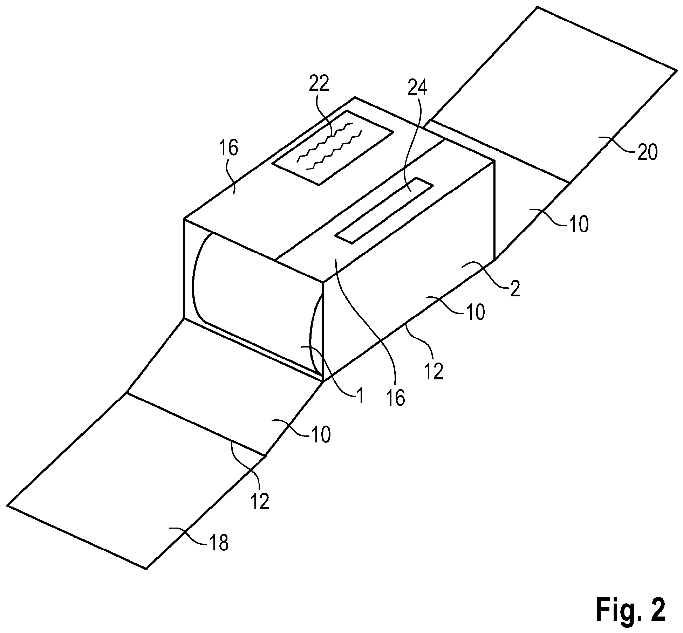

Zur weiteren Verdeutlichung ist in FIG. 2 das bereits teilweise von der Umhüllung 2 umgebene Hygieneprodukt 1 gezeigt; in der dargestellten Situation sind die Seitenlaschen 16 unter Ausbildung der vorgesehenen, an die Produktdicke angepassten Seitenkanten 10 bereits umgefaltet und aneinander fixiert, wohingegen die Umfaltung der vorder- und rückseitigen Laschen 18, 20 noch aussteht. Wie dieser Darstellung ebenfalls entnehmbar ist, ist der Umhüllungsrohling mit einer Bedruckung in Form eines Motivs 22 und mit einem Fensterausschnitt 24 versehen.

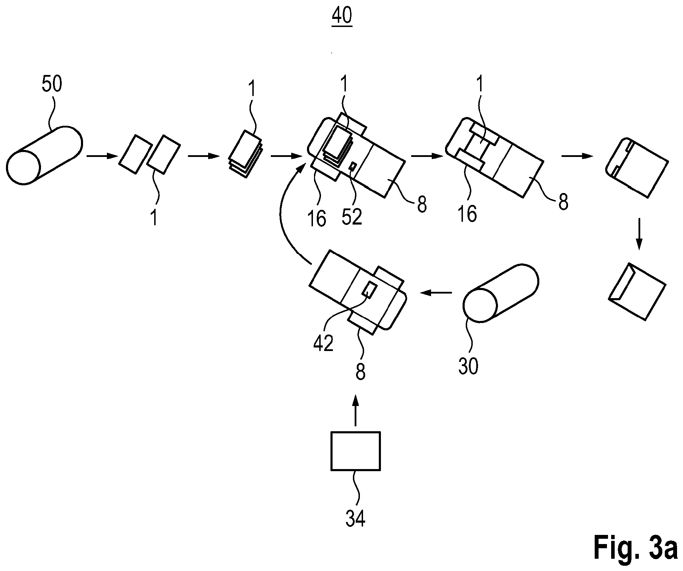

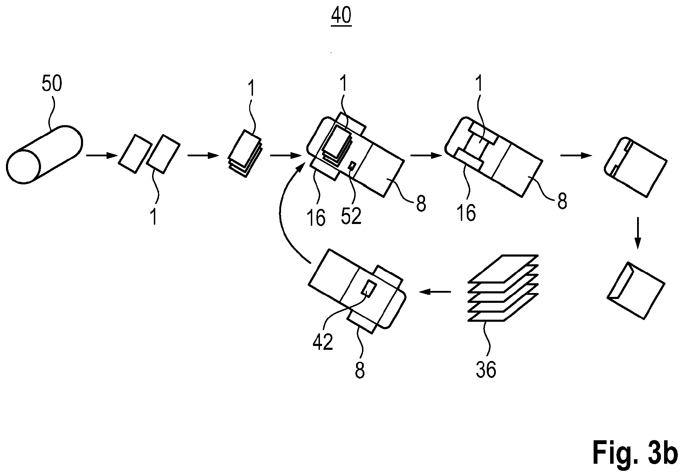

Der Verfahrensablauf beim Umhüllen des Hygieneprodukts 1 ist in FIG. 3 schematisch dargestellt. **In** der ersten, in FIG. 3a dargestellten Variante werden die Umhüllungsrohlinge 8 über eine erste Materialbahn 30 bereitgestellt. **In** einem ersten Verfahrensschritt wird die Materialbahn 30 mittels einer Schneideinheit 34 in der Art einer Vereinzelung in die Umhüllungsrohlinge 8 geschnitten; alternativ können die Umhüllungsrohlinge 8 gemäß der in FIG. 3b gezeigten Variante aber auch bereits vorgeschnitten oder als Einzelblatt oder Bogen bereitgestellt werden. Mittels der Schneideinheit 34 ist ein besonders individueller Zuschnitt möglich, der auch von Umhüllungsrohling 8 zu Umhüllungsrohling 8 durch eine entsprechende Steuerung der Schneideinheit 34 geändert werden kann. Somit ist eine direkte und optimale Anpassung an das zu verpackende Hygieneprodukt 1 möglich. Sollte die Materialbahn 30 und somit auch der Umhüllungsrohling 8 bereits vollständig bedruckt sein, wird der Umhüllungsrohling 8 im nächsten Verfahrensschritt der Umhüllungseinheit 40 zugeführt. Ansonsten kann der Umhüllungsrohling 8 in einem nicht dargestellten Verfahrensschritt durch eine nicht dargestellte Druckeinheit vor der Zuführung zur Umhüllungseinheit 40 individuell und einzeln bedruckt werden. Zusätzlich oder in alternativer Ausführung ist es aber auch möglich, dass durch eine weitere Druckeinheit bereits auf die Materialbahn 30 gedruckt wird. Dies hat den Vorteil, dass über den gesamten späteren Zuschnitt gedruckt werden kann und somit keine systembedingten druckfreien Ränder entstehen. Durch die Verwendung von zwei nach einander geschalteten Druckeinheiten ist es darüber hinaus ebenfalls möglich die Verpackung sowohl beidseitig außen und auch innen zu bedrucken. Alternativ oder zusätzlich zu einer solchen Bedruckung kann auch die Prägung eines Motivs und/oder die Einbringung einer Perforation vorgesehen sein.

Es ist ebenfalls möglich, dass in einem weiteren, der Zuführung zur Umhüllungseinheit 40 vorgelagerten Verfahrensschritt oder auch zusammen mit dem Zuschnitt des Umhüllungsrohlings 8 ein Fensterelement 42 in den Umhüllungsrohling 8 geschnitten wird und dieses mit einer Fensterfolie verschlossen wird. Dadurch ist es möglich auch Umhüllungen 2 mit einem Sichtfenster herzustellen.

Die schematische Darstellung des Verfahrens nach FIG. 3 zeigt darüber hinaus eine zweite Materialbahn 50 für die Herstellung des zu verpackenden Hygieneprodukts 1. Diese zweite Materialbahn 50 wird in an sich bekannter Weise verarbeitet, wobei die Hygieneprodukte 1 durch Schneiden, ggf. Falten, ggf. Stapelbildung, je nach vorgegebenen Anforderungen, bereitgestellt werden. Das fertige Produkt 1, ggf. als Produktstapel, wird anschließend der Umhüllungseinheit 40 zugeführt.

Für eine besonders zuverlässige und genaue Positionierung werden der Hygieneartikel 1 einerseits und der zugeordnete Umhüllungsrohling 8 andererseits getaktet und zueinander synchronisiert der Umhüllungsstation 40 zugeführt, wobei die Taktung oder Synchronisierung von Hygieneartikel 1 einerseits und zugeordnetem Umhüllungsrohling 8 andererseits durch Abfragen einer Druckmarke 52 überwacht wird. Bei der Vereinigung des Hygieneprodukts 1 mit dem Umhüllungsrohling 8 werden beide in einer Linie und gleichläufig zueinander transportiert. Dadurch werden Relativbewegungen zwischen den beiden vermieden, und das Produkt 1 kann auf besonders einfache Art und Weise, beispielsweise durch Ablage, auf dem Umhüllungsrohling 8 positionsgenau platziert werden. Somit liegt das zu verpackende Produkt 1 in der Umhüllungseinheit 40 auf der Innenseite des Umhüllungsrohlings 8.

**In** einem ersten Umhüllungsschritt werden zunächst die Seitenklappen 16 des Umhüllungsrohlings 8 gefaltet und um das Hygieneprodukt 1 geschlagen. Gleichzeitig oder in einem nachgeschalteten Verfahrensschritt wird im Ausführungsbeispiel die Außenseite der Seitenklappen 16 mit einem Klebefilm überzogen, um das anschließende Fixieren zu ermöglichen. In einem zweiten Umhüllungsvorgang werden anschließend die vorder- und rückseitigen Klappen 18, 20 gefaltet, um das Produkt 1 geschlagen und mit den Seitenklappen 16 verklebt.

Zur Fixierung ist im Ausführungsbeispiel ein Klebevorgang vorgesehen; alternativ oder zusätzlich kann die Fixierung aber auch mittels adhäsivem Fixiermaterial, z. B. Heißleim, Kaltleim, selbstklebendes Tape oder andere, mittels mechanischem Fixiermaterial, z. B. Klettverschluss, Druckknopf oder andere, mittels thermoplastischem Fixiermaterial, z. B. heiß aktivierbarer Kaltleim, Wachs, Lack oder andere, oder ohne Fixiermaterial, z. B. durch rein mechanisches oder thermomechanisches Verprägen, Ultraschall, Einschieben der Laschen in dafür vorgesehene Schlitze oder andere oder mittels einer Kombination aus einer oder mehrerer dieser Möglichkeiten erfolgen.

Die Fixiermaterialien können vollflächig oder in Teilbereichen aufgebracht werden, entweder bereits durch den Materialhersteller, oder inline in der Maschine. Dort z. B. durch Drucken, Sprühen, oder andere für das betreffende Material geeignete Verfahren. Insbesondere können die Laschen 16, 18, 20 der Umhüllung 2 in oder nach der Umhüllungsstation 40 thermisch und/oder mechanisch und/oder durch Kleben, vorzugsweise wiederverschließbar, aneinander fixiert werden.

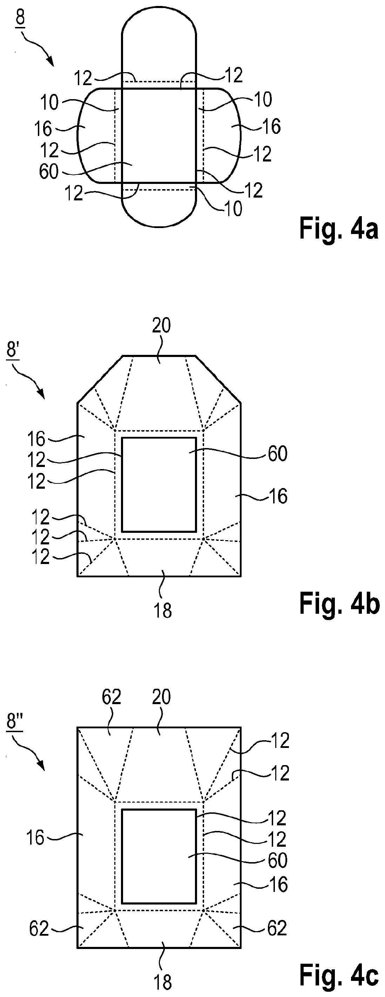

In Fig. 4 sind einige Varianten eines vorgeschnittenen und mit Falzkanten 12 versehenen Umhüllungsrohlings 8 dargestellt, wie sie in den vorstehend beschriebenen Einrichtungen zum Einsatz kommen könnten. In Fig. 4a ist dabei ein Umhüllungsrohling 8 dargestellt, der zur Bildung einer Umhüllung 2 in Art und Form eines Briefumschlags herangezogen werden könnte. Hierbei sind die vier inneren Falzkanten 12 Rechteck-artig um die Basisfläche 60 der Umhüllung 2 herum angeordnet. Jeweils nach außen versetzt und parallel zur inneren Falzkante 12 ist dann eine zugeordnete äußere Falzkante 12 vorgesehen, so dass jeweils zwischen innerer und äußerer Falzkante 12 die Seitenflächen oder Seitenlaschen 10 der Umhüllung 2 ausgebildet werden. Ein derartig ausgestalteter Umhüllungsrohling 8 kann in dem oben beschriebenen Faltprozess vergleichsweise einfach und zuverlässig verarbeitet werden, wobei der zu verpackende Hygieneartikel vergleichbar zu einem Briefumschlag in die Umhüllung 2 eingeschlagen wird.

In einem weiter entwickelten und als eigenständig erfinderisch angesehenen Umhüllungskonzept ist der Umhüllungsrohling 8 demgegenüber aber auch noch für ein vollständiges Umschließen des Hygieneartikels ausgelegt. Insbesondere ist dabei berücksichtigt, dass der in Fig. 4a dargestellte Umhüllungsrohling 8 nach der Faltung im Bereich der sich bildenden Ecken in geringem Umfang Öffnungen freilassen könnte. Dies könnte die Aufbewahrung feucht gehaltener Produkte erschweren, oder andererseits auch die trockene Aufbewahrung der Hygieneprodukte in feuchter Außenumgebung behindern.

Um dem entgegenzuwirken, sind in den als eigenständig erfinderisch angesehenen Ausführungsformen des Umhüllungsrohlings 8',8" gem. Fig. 4b und Fig. 4c die Falzkanten 12 derart positioniert, dass nach der Faltung in der sich ausbildenden Umhüllung 2 deren Ecken durch eigenständige Materialsegmente vollständig überdeckt werden. Dazu ist in diesen Ausgestaltungen ebenfalls eine Anzahl innerer Falzkanten 12 vorgesehen, die Rechteck-artig um die Basisfläche 60 der Umhüllung 2 herum angeordnet sind. In diesen Ausführungsformen befindet sich aber zwischen den Seitenlaschen 16 und der jeweils benachbarten vorder- oder rückseitigen Lasche 18, 20 noch eine Anzahl weiterer Materialsegmente 62, die bei der Faltung über die sich ausbildenden Ecken gefaltet werden. Im Vergleich zu gängigen Produkten entspricht das dabei entstehende Faltmuster in etwa dem einer Verpackung einer Schokoladentafel.

Der Umhüllungsrohling 8',8" kann in diesen Varianten, ausgehend von einem zunächst rechteckigen Vorschnitt, unterschiedlich konturiert und zugeschnitten sein, abhängig vom individuell vorgegebenen Einsatzzweck. In Fig. 4b ist dazu ein Beispiel mit leicht zugeschnittenem oberen Bereich gezeigt, wobei der entstehende Verschnitt (d. h. der beim Zuschnitt aus einem rechteckigen Rohling entstehende Abfall) bereits vergleichsweise gering gehalten werden kann. Eine vollständige Vermeidung von Verschnitt und damit Abfall ist demgegenüber durch den Umhüllungsrohling 8" ermöglicht, wie er in Fig. 4c dargestellt ist. Bei diesem kann die gesamte Grundfläche des ursprünglich rechteckigen Rohlings zur Bildung der die Umhüllung 2 bildenden Teilflächen genutzt werden.

### Bezugszeichenliste

- 1: Hygieneprodukt
- 2: Umhüllung
- 4, 6: Pfeil
- 8,8',8": Umhüllungsrohling
- 10: Seitenkante
- 12: Falzkante
- 14: Pfeil
- 15: Druckmarke
- 16: Seitenlasche
- 18: vorderseitige Lasche
- 20: rückseitige Lasche
- 22: Motiv
- 24: Fensterausschnitt
- 30: Materialbahn
- 34: Schneideinheit
- 36: Einzelblatt
- 40: Umhüllungseinheit
- 42: Fensterelement
- 50: Materialbahn
- 52: Druckmarke
- 60: Basisfläche
- 62: Materialsegment

## Patentansprüche

1. Verfahren zum Umhüllen eines Hygieneartikels (1) oder eines Stapels aus einer Anzahl von Hygieneartikeln (1), nämlich von absorbierenden Einwegartikeln zur Aufnahme von Körperausscheidungen, wobei der Hygieneartikel (1) oder der Stapel aus Hygieneartikeln (1) mit einer aus einem nachhaltigen, insbesondere biologisch abbaubaren und/oder recycelbaren, Rohstoff aus der Gruppe umfassend Papier, Kraftpapier, fettdichtes und/oder nassfestes Papier, Filterpapier, Krepp-Papier, Pergamid, Eukalyptus, Tissue, Bambus, Papyrus, Hanf, Filz, Wolle, Baumwolle, Leinen gebildeten Umhüllung (2) versehen wird, bei dem
a. der Hygieneartikel (1) bzw. der Stapel aus Hygieneartikeln (1) mittels einer ersten Zuführeinheit einer Umhüllungsstation (40) zugeführt wird,
b. ein aus dem nachhaltigen Rohstoff gebildeter Umhüllungsrohling (8) in einer Vorrill-Station mit zur Bildung von einer Anzahl von Seitenkanten (10) oder Seitenflächen der Umhüllung (2) geeigneten Falzkanten (12) versehen und anschließend mittels einer zweiten Zuführeinheit der Umhüllungsstation (40) zugeführt wird,
c. in der Umhüllungsstation (40) der Hygieneartikel (1) bzw. der Stapel aus Hygieneartikeln (1) in einer vorgegebenen Sollposition auf dem Umhüllungsrohling (8) positioniert wird, und
d. in der Umhüllungsstation (40) der Hygieneartikel (1) bzw. der Stapel aus Hygieneartikeln (1) durch Faltung des Umhüllungsrohlings (8) und anschließendes Verschließen umhüllt wird, indem
d1. Seitenlaschen (16) unter Ausbildung der durch die Falzkanten (12) vordefinierten Seitenkanten (10) eingefaltet werden,
d2. die vorder- und rückseitigen Laschen (18, 20) unter Ausbildung der durch die für diese vorgesehenen Falzkanten (12) vordefinierten Seitenkanten (10) eingefaltet werden, und
d3. die vorder- und rückseitigen Laschen (18, 20) an den Seitenlaschen (16) und aneinander fixiert werden,
wobei der Umhüllungsrohling (8) für ein vollständiges Umschließen des Hygieneartikels (1) ausgelegt ist, und wobei sich zwischen den Seitenlaschen (16) und der jeweils benachbarten vorder- oder rückseitigen Lasche (18, 20) noch eine Anzahl weiterer Materialsegmente (62) befindet, die bei der Faltung über die sich ausbildenden Ecken gefaltet werden.

2. Verfahren nach Anspruch 1, bei dem der Umhüllungsrohling (8) vor der Zuführung zur Umhüllungsstation (40) aus einer Endlosbahn (30) des Umhüllungsrohstoffs vereinzelt wird.

3. Verfahren nach Anspruch 1 oder 2, bei dem die Seitenkanten (10) des Umhüllungsrohlings (8) vor oder in der Umhüllungsstation (40) zugeschnitten werden.

4. Verfahren nach einem der Ansprüche 1 bis 3, bei dem die Laschen (16, 18, 20) der Umhüllung (2) in oder nach der Umhüllungsstation (40) thermisch und/oder mechanisch und/oder durch Kleben, vorzugsweise wiederverschließbar, aneinander fixiert werden.

5. Verfahren nach einem der Ansprüche 1 bis 4, bei dem der Umhüllungsrohling (8) mit einer Bedruckung versehen wird.

6. Verfahren nach einem der Ansprüche 1 bis 5, bei dem der Umhüllungsrohling (8) mit einer Prägung versehen wird.

7. Verfahren nach einem der Ansprüche 1 bis 6, bei dem der Umhüllungsrohling (8) mit einer Perforierung oder Perforation versehen wird.

8. Verfahren nach einem der Ansprüche 1 bis 7, bei dem beim Umhüllungsrohling ein Fenster (42) ausgeschnitten wird, das anschließend durch Einkleben mit einer Fensterfolie verschlossen wird.

9. Verfahren nach einem der Ansprüche 1 bis 8, bei dem der Hygieneartikel (1) bzw. der Stapel aus Hygieneartikeln (1) einerseits und ein zugeordneter Umhüllungsrohling (8) andererseits getaktet und zueinander synchronisiert der Umhüllungsstation (40) zugeführt werden.

10. Verfahren nach Anspruch 9, bei dem die Taktung oder Synchronisierung von Hygieneartikel (1) bzw. Stapel aus Hygieneartikeln (1) einerseits und zugeordnetem Umhüllungsrohling (8) andererseits durch Abfragen einer Druckmarke (15, 52) überwacht wird.

11. Verfahren nach einem der Ansprüche 1 bis 10, bei dem der zur Bildung der Umhüllung (2) vorgesehene Rohstoff mehrlagig, vorzugsweise als innen kaschierte Papierbahn, ausgeführt ist.

12. Verfahren nach einem der Ansprüche 1 bis 11, bei der der oder die Hygieneartikel (1) nach dem Einfalten in die Umhüllung (2) an dieser fixiert werden.

## Claims

1. A method for wrapping a hygiene product (1) or a stack comprised of a number of hygiene products (1), specifically of absorbent disposable products for absorbing bodily excretions, wherein the hygiene product (1) or the stack of hygiene products (1) is provided with a wrapping (2) formed out of a sustainable, in particular biodegradable and/or recyclable, raw material from the group comprised of paper, kraft paper, greaseproof and/or waterproof paper, filter paper, crepe paper, parchment, eucalyptus, tissue, bamboo, papyrus, hemp, felt, wool, cotton, linen, in which
a. the hygiene product (1) or the stack comprised hygiene articles (1) is supplied to a first supply unit of a wrapping station (40),
b. a wrapping blank (8) formed out of the sustainable raw material is provided in a presorting station with fold edges (12) suitable for forming a number of side edges (10) or side surfaces of the wrapping (2), and then supplied to the wrapping station (40) by means of a second supply unit,
c. the hygiene product (1) or the stack of hygiene products (1) at the wrapping station (40) is positioned in a defined set position on the wrapping blank (8), and
d. the hygiene product (1) or the stack of hygiene products (1) at the wrapping station (40) is wrapped by folding the wrapping blank (8) and subsequent sealing, in that
d1. side tabs (16) for forming the side edges (10)
predefined by the fold edges (12) are folded in,
d2. the front and rear tabs (18, 20) are folded in, forming the side edges (10) predefined by the fold edges (12) and provided for them, and
d3. the front and rear tabs (18, 20) are fixed to the side tabs (16) and to each other,
wherein the wrapping blank (8) is designed to completely envelop the hygiene product (1), and wherein a number of additional material segments (62) are located between the side tabs (16) and the respective adjacent front or rear tab (18, 20), which are folded while being folded over the corners that form.

2. The method according to claim 1, in which the wrapping blank (8) is separated from a continuous web (30) of the wrapping material before supplied to the wrapping station (40).

3. The method according to claim 1 or 2, in which the side edges (10) of the wrapping material (8) are cut to size before or in the wrapping station (40).

4. The method according to one of claims 1 to 3, in which the tabs (16, 18, 20) of the wrapping (2) are thermally and/or mechanically and/or adhesively, preferably resealably, fixed to each other in or after the wrapping station (40).

5. The method according to one of claims 1 to 4, in which the wrapping material (8) is printed.

6. The method according to one of claims 1 to 5, in which the wrapping material (8) is embossed.

7. The method according to one of claims 1 to 6, in which the wrapping material (8) is perforated or punched.

8. The method according to one of claims 1 to 7, in which a window (42) is cut out of the wrapping material, and subsequently sealed by sticking on window film.

9. The method according to one of claims 1 to 8, in which the hygiene product (1) or the stack of hygiene products (1) on the one hand and an allocated wrapping material (8) on the other are supplied to the wrapping station (40) timed and synchronized with each other.

10. The method according to claim 9, in which the timing or synchronizing of hygiene products (1) or stacks of hygiene products (1) on the one hand and allocated wrapping material (8) on the other is monitored by checking a print mark (15, 52).

11. The method according to one of claims 1 to 10, in which the material provided to form the wrapping (2) is designed in multiple layers, preferably as a paper web laminated on the inside.

12. The method according to one of claims 1 to 11, in which the hygiene product(s) (1) is/are fixed to the wrapping (2) after folded into the latter.

## Revendications

1. Procédé de conditionnement d'un article d'hygiène (1) ou d'une pile d'un certain nombre d'articles d'hygiène (1), à savoir d'articles absorbants jetables destinés à absorber les excrétions corporelles, l'article d'hygiène (1) ou la pile d'articles d'hygiène (1) étant revêtus d'une matière première durable, notamment biodégradable et/ou recyclable, comprenant du papier, du papier kraft, du papier gras et/ou résistant à l'humidité, du papier filtre, du papier crêpe, du pargamide, de l'eucalyptus, du papier essuie-tout, du bambou, du papyrus, du chanvre, du feutre, de la laine, du coton, une enveloppe constituée de lin (2),
a. l'article d'hygiène (1) ou la pile d'articles d'hygiène (1) étant acheminés vers une station de conditionnement (40) au moyen d'une première unité d'alimentation,
b. une matière première de conditionnement (8) issue de la matière première durable étant munie, dans une station de pré-rainurage, de bords pliants (12) adaptés à la formation d'un certain nombre de bords latéraux (10) ou de surfaces latérales du conditionnement (2), puis amenée à la station de conditionnement (40) au moyen d'une seconde unité d'alimentation ;
c. dans le poste de conditionnement (40), l'article d'hygiène (1) ou la pile d'articles d'hygiène (1) étant positionné dans une position de consigne prédéterminée sur le conditionnement brut (8), et
d. dans la station de conditionnement (40), les articles d'hygiène (1) ou la pile d'articles d'hygiène (1) étant enveloppés par pliage du conditionnement brut (8) et par fermeture ultérieure, par le ; fait que
d1. les languettes latérales (16) sont pliées en formant les bords latéraux (10) prédéfinis par les bords de pliage (12),
d2. les languettes avant et arrière (18, 20) sont pliées en formant les bords latéraux (10) prédéfinis pour ceux-ci (12), et
d3. les languettes avant et arrière (18, 20) sont fixées sur les languettes latérales (16) ;
et le conditionnement brut (8) est conçu pour entourer complètement l'article d'hygiène (1) et, entre les languettes latérales (16) et la languette frontale ou arrière respectivement adjacente (18, 20), il y ayant encore un certain nombre d'autres segments de matériau (62) qui sont pliés lors du pliage sur les coins en formation.

2. Procédé selon la revendication 1, dans lequel le conditionnement brut (8) est séparé dans une bande continue (30) du conditionnement brut avant d'être amené à la station de conditionnement (40).

3. Procédé selon la revendication 1 ou 2, dans lequel les bords latéraux (10) du conditionnement brut (8) sont découpés en amont ou dans la station de conditionnement (40).

4. Procédé selon l'une des revendications 1 à 3, dans lequel les languettes (16, 18, 20) du conditionnement (2) sont fixées les unes aux autres dans ou après la station de conditionnement (40), de préférence de manière à être refermables, par des moyens thermiques et/ou mécaniques et/ou par collage

5. Procédé selon l'une des revendications 1 à 4, au cours duquel le conditionnement brut (8) est imprimé.

6. Procédé selon l'une des revendications 1 à 5, au cours duquel le conditionnement brut (8) est pourvu d'un gaufrage.

7. Procédé selon l'une des revendications 1 à 6, au cours duquel le conditionnement brut (8) est pourvu d'une perforation ou de trous.

8. Procédé selon l'une des revendications 1 à 7, au cours duquel une fenêtre (42) est découpée dans le conditionnement brut, laquelle fenêtre est ensuite fermée par collage avec un film de fenêtre.

9. Procédé selon l'une des revendications 1 à 8, dans lequel l'article d'hygiène (1) ou la pile d'articles d'hygiène (1) d'une part, et un conditionnement brut (8) d'autre part sont acheminés de manière cadencée et synchronisée mutuellement vers la station de conditionnement (40).

10. Procédé selon la revendication 9, dans laquelle le cadencement ou la synchronisation des articles d'hygiène (1) ou des piles d'articles d'hygiène (1) d'une part, et du conditionnement brut associé (8) d'autre part, est surveillé en consultant une marque d'impression (15, 52).

11. Procédé selon l'une des revendications 1 à 10, dans lequel la matière première prévue pour la formation du conditionnement (2) est réalisée en plusieurs couches, de préférence sous forme d'une bande de papier doublée à l'intérieur.

12. Procédé selon l'une des revendications 1 à 11, dans lequel le ou les articles d'hygiène (1), après pliage dans le conditionnement, sont fixés sur celui-ci (2).
